# EUROPEAN PATENT APPLICATION

(11) **EP 3 922 170 A1**
(43) Date of publication of application: **15.12.2021**
(21) Application number: 21162311.1
(22) Date of filing: 12.03.2021
(51) Int. Cl.: A61B 5/00

(54) **A NEW MODEL FOR EVALUATING WOUND HEALING IN HUMANS**

(30) Priority: 13.03.2020 EP 20163018
(71) Applicant: Medice Arzneimittel Pütter GmbH & Co. KG, 58638 Iserlohn (DE)
(72) Inventor: AMMER, Richard, 58644 Iserlohn (DE)
(74) Representative: Isarpatent

(57) **Abstract**

A novel wound model for acute and non-acute wounds affecting all skin layers (2) to evaluate and to compare the wound healing properties of different topical wound healing approaches versus standard treatment (3) in patients undergoing catheter de-placement after having undergone placement of Sheldon multi-lumen catheter (11 French diameter) in the external jugular vein at the neck by Seldinger technique (4) to assess wound healing after few hours or after up to 14 (+/- 2) days after withdrawal of catheter.

## Description

### Technical field

The present invention is related to a method for evaluating topical approaches applied on acute and none-acute wounds for their wound healing properties.

### Background

Skin lesions affecting all layers of the skin (epidermis-dermis and basal membrane) can have multiple and various causes derived from heterogeneous etiology such as acute, e.g. accidental trauma, intentional incisions, or chronic mainly due to an underlying disease and imbalanced physiological environment needed for re-epithelization.

Established wound models offer multiple advantages in assessing wound healing properties by different approaches. Such models allow standardized skin lesions which are comparable in size, surface area and wound depth. These techniques can generate standardized wounds of nearly identical size accurately reproduced by the same mode of action by same conditions enabling standardized comparisons and allowing representative conclusions in a limited number of patients.

Such categories of models are
1. abrasive, affecting non-vascularized epidermis and opening capillary loops of dermal papillae leading to punctual bleeding [10], or
2. non-invasive, such as measuring transepidermal water loss measurements (TEWL) in combination with tape stripping or suction blister wounds [17, 21, 21], or
3. invasive, involving first-degree burns, laser, abrasion by using brushes or sandpaper and partial-thickness excisions or microdermabrasion by using a dermatome or
4. invasive, such as mini-incisions, biopsy (punch or scalpel) affecting epidermis and dermis involving [11, 22].

In these models, physiological wound healing conditions are considered comparable for acute wounds without a special reference to the wounding mechanism (such as cuts, lacerations or superficial wounds) and results are considered assignable [11]. Therefore, it has to be pointed on the fact that, according to the affected tissues, results can differ in between the different models. In consequence, our model fits to category 4 with minor incisions through all three layers of the skin in a routine clinical procedure.

### Summary of the invention

The present invention offers a novel method for evaluating topical approaches applied on acute and none-acute wounds for their wound healing properties. The method provides a routine approach with standardized incisions set and utilized as acute or non-acute wound model, wherein the healing properties and relative wound healing rate of a topical wound healing approach in comparison to traditional or standard approaches as control (traditional treatment) as well as the outcome of acute and non-acute wounds treated with the different approaches can be investigated.

Further aspects and embodiments of the invention are disclosed in the dependent claims and can be taken from the following description, figures and examples, without being limited thereto.

### Figures

The enclosed drawings should illustrate embodiments of the present invention and convey a further understanding thereof. In connection with the description they serve as explanation of concepts and principles of the invention. Other embodiments and many of the stated advantages can be derived in relation to the drawings. The elements of the drawings are not necessarily to scale towards each other. Identical, functionally equivalent and acting equal features and components are denoted in the figures of the drawings with the same reference numbers, unless noted otherwise.

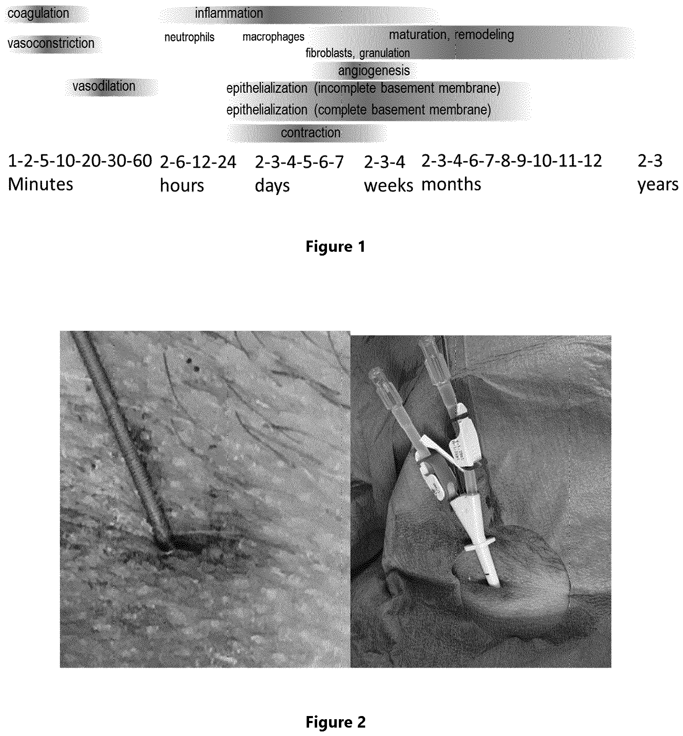

Figure 1: Wound types. Acute versus non-acute and chronic wounds follow several stages of healing (modified from Martin P, Nunan R (2015).
Figure 2: Representative images of a standardized incision into the jugular vein in one subject at baseline (day 0).

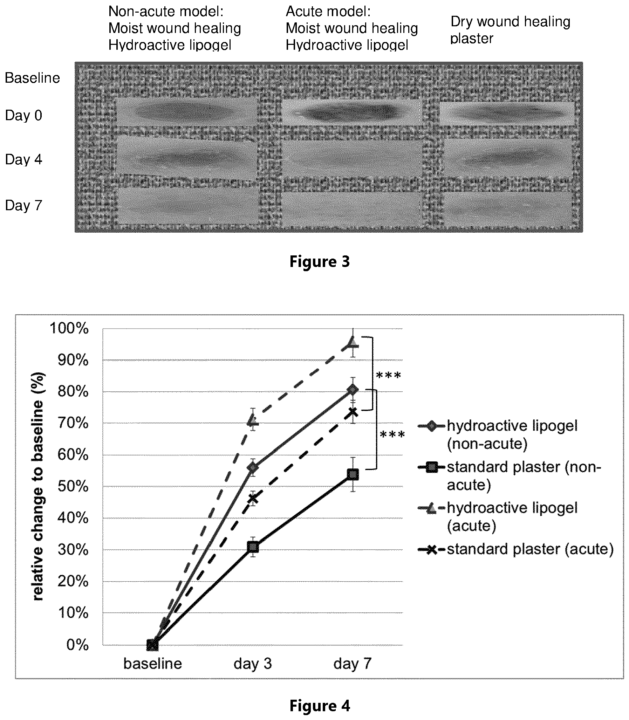

Figure 3: Representative video microscope images, documenting the healing progress of the three standardized lesions of patients at day 0 (baseline), day 3 and day 7 treated with the investigational product versus the reference standard plaster in acute (5±1 hour old incision) versus non-acute (14±2 days old incision) model.
Figure 4: Exemplary line graph of mean open wound area by treatment against time for raw data. All assessment time points were significantly different for investigational product (hydroactive lipogel, in pink) vs. traditional treatment (standard plaster, in blue) in both wound types (acute = 4-6 hours, dashed lines) (p <0.01) and non-acute (14 ± 2 days, solid lines) (p <0.001).

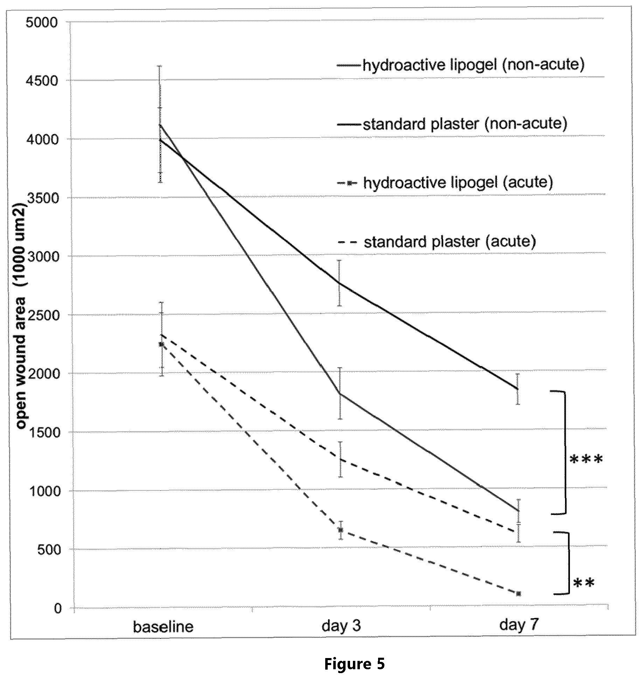

Figure 5: Exemplary line graph of mean open wound area/crusts by treatment against time for relative difference to baseline. All assessment time points were significantly different for product (hydroactive lipogel, in pink) vs. traditional treatment (standard plaster, in blue) in both wound types (acute = 4-6 hours, dashed lines) (p <0.001) and non-acute (14 ± 2 days, solid lines) (p <0.001).

### Description of invention

### Definitions

Unless defined otherwise, technical and scientific terms used herein have the same meaning as commonly understood by one of ordinary skill in the art to which this invention belongs.

Amounts within the present invention are given in wt.%, unless stated otherwise or clear from context.

Quantitative image analysis in the context of the present invention is not particularly limited. As is generally understood, quantitative image analysis involves utilizing digital images to provide data and information. This is done with computer technology to recognize patterns, create maps, and process signals within images.

A plaster in the context of the present invention is not particularly limited. As is generally understood, a plaster can be any kind of standard plaster or dressing which is used to cover a wound. The plaster or dressing may or may not be held in place by a bandage.

A lipogel in the context of the present invention is not particularly limited. As is generally understood, a lipogel is used to cover a wound. According to certain embodiments, the lipogel is a hydroactive lipogel. The lipogel may be a wax ointment.

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

The present invention relates to a novel model for evaluating wound healing in humans. A wound model is provided comprising venous access by Seldinger technique and digital photography and photo analysis enabling assessment of wound healing properties of different topical approaches applied.

The invention relates to a method for evaluating topical approaches applied on acute and none-acute wounds for their wound healing properties. The method comprises a first step of evaluating a wound and a second step of evaluating the wound after one to three days after the first step and after four to seven days after the first step and optionally after eight to fourteen days after the first step. In certain embodiments, the wound is evaluated after three days and after seven days after the first step. The evaluation of the wound includes taking at least one digital photograph of the wound. In some embodiments, a topical approach was applied to the wound during the time between each evaluation. Further, the method comprises a third step of analyzing the photographs taken in the first and second step by quantitative image analysis, wherein the quantitative image analysis includes determining the mean open wound area of the wound.

According to certain embodiments, the method for evaluating topical approaches on wound healing properties can further comprise a fourth step of analyzing wound healing by relating the mean open wound area to the time of each evaluation of the wound in the first and second step. The fourth step can further include calculating a ratio of the mean open wound area of the wound of the evaluation of the first step to the mean open wound area of the wound of each evaluation of the wound in the first and second step.

According to some embodiments, the wound is an incision affecting all skin layers. The skin layers include epidermis, dermis, and subcutis. In certain embodiments, the incision is made using a stylet. The stylet may be a stylet size 11 (1 to 6.67 mm). In some embodiments, the incision is a minor incision.

In certain embodiments, the wound is caused by the procedure of a placement of a catheter, wherein in the first step of the method, the wound is evaluated after displacement of the catheter. According to certain embodiments, the catheter is placed using Seldinger technique. In some embodiments, the catheter is placed in the left or right external jungular vein in the neck.

The catheter may be placed after skin disinfection of the neck with a standard disinfectant such as octenidine phenoxyethanol. The Seldinger technique used for the placement of the catheter is described in the following. The patient or subject is punctured with a sharp hollow needle assisted by or without ultrasound guidance. A round-tipped guidewire is advanced through the lumen of the needle, the needle is withdrawn. A minor incision through all three layers of the skin is made using a stylet size 11 (1 to 6.67 mm). Subsequently, a sheath is passed over the guidewire into the vein in order to enlarge the incision bluntly. After passing the multi-lumen Sheldon catheter, the guidewire is withdrawn (Figure 2).

The Seldinger technique leads to bloody skin incision affecting epidermis, dermis, and subcutis, adventitia tissue and puncture of jugular vein.

According to certain embodiments, the wound is an acute wound caused by a cut conducted for the placement of a catheter, wherein the catheter is displaced after 1 to 24 hours, preferably after 2 to 12 hours after its placement. In preferred embodiments, the catheter is displaced after 4 to 6 hours after its placement. These incisions are classified as acute wounds where coagulation, vasocontraction, dilatation have taken place and inflammation (with the migration of neutrophils) has been initiated (Figure 1).

According to some embodiments, the wound is a non-acute wound caused by a cut conducted for the placement of a catheter, wherein the catheter is displaced after 2 to 20 days, preferably after 6 to 18 days, more preferably after 12 to 16 days after its placement. According to preferred embodiments, the catheter is displaced after 14 days after its placement. In these wounds, the process of wound healing has advanced with progressed inflammation, migration of macrophages, granulation formation and remodeling initiated by fibroblasts. These incisions are classified as non-acute (Figure 1).

In some embodiments, no sealing device is used to close the incision, but standard manual compression for a few minutes is applied when the catheter has been displaced. After photo documentation of the incision (baseline, day 0), the incision is covered solely by the standard or by the investigational product covered by the identical type of standard plaster.

In certain embodiments, the catheter is an external multi-lumen catheter, preferably a Sheldon multi-lumen catheter with 1 mm to 6.67 mm diameter. According to preferred embodiments, the catheter is a Sheldon multi-lumen catheter with 3.67 mm diameter.

According to some embodiments, the topical approach corresponds to any one of covering the wound with a plaster and/or covering the wound with a lipogel.

In some embodiments, the evaluation of the wound further includes the assessment of inflammation/infection regarding at least one of erythema, pain, odor, non-healing, excessive exudation and hyperthermia. Alternatively or in addition, the evaluation of the wound further includes the assessment of allergic reactions regarding erythema, edema and itching.

According to certain embodiments, the area captured by the digital photographs is 0.3 to 20 cm in diameter. In some embodiments, the mean open wound area is determined by digital microscopy, preferably including a histological image analysis program for measuring wound areas.

In certain embodiments, the evaluation comprises a comparison of the wound healing properties of an investigational product versus a standard product (for example a standard plaster). The comparison relates to the wound area assessed by quantitative image analysis at day 3 and day 7, quantified as area under the curve (AUC) and relative differences to baseline over time.

The above embodiments can be combined arbitrarily, if appropriate. Further possible embodiments and implementations of the invention comprise also combinations of features not explicitly mentioned in the foregoing or in the following with regard to the Examples of the invention. Particularly, a person skilled in the art will also add individual aspects as improvements or additions to the respective basic form of the invention.

### Examples

After skin disinfection of the neck with a standard disinfectant (octenidine phenoxyethanol), the right or left external jugular vein (of recruited humans (e.g. patients with acute or chronic renal insufficiency in need for vein access via an external multi-lumen catheter (Sheldon with 11 (3 to 20) French in diameter) placed by modified Seldinger technique) is punctured with a sharp hollow needle assisted by or without ultrasound guidance. A round-tipped guidewire is advanced through the lumen of the needle, the needle is withdrawn. A minor incision through all three layers of the skin is made using a stylet size 11 (3 to 20 F). Subsequently, a sheath is passed over the guidewire into the vein in order to enlarge the incision bluntly. After passing the multi-lumen Sheldon catheter, the guidewire is withdrawn (Figure 2).

When the Sheldon catheter has been displaced, no sealing device is used to close the incision, but standard manual compression for a few minutes is applied. After photo documentation of incision (baseline, day 0), patients are assigned to have the incision covered solely by standard or by investigational product covered by the identical type of standard plaster. Digital photos are also obtained at certain time points (e.g. day 3 and 7). Visual scoring and measurements of wound area is performed by digital microscopy, a histological image analysis program for measuring wound areas by an observer.

Seldinger technique applied [13, 14] leads to bloody skin incision affecting epidermis, dermis, and subcutis, adventitia tissue and puncture of jugular vein (Figure 2). If catheter was installed only for one session, then catheter is displaced approx. 4 to 6 hours after initial incision. These incisions were classified as acute wounds where coagulation, vasocontraction, dilatation have taken place and inflammation (with the migration of neutrophils) has been initiated (Figure 1). If the catheter is placed for several dialysis sessions and kept for two weeks (14 ± 2 days), the process of wound healing has advanced with progressed inflammation, migration of macrophages, granulation formation and remodeling initiated by fibroblasts. These incisions are classified as non-acute (Figure 1).

At certain time points (e.g. day 0, 3, 7, 14) digital photographs of the wounds are taken. Image acquisition is performed with a handheld camera, ideally with a tubular distance holder (complete extinction of light) to enable reproducible images with defined magnification. The captured area is 0.3 to 20 cm in diameter. Pictures are taken before applying investigational products or plaster. Afterwards the open wound area is marked by an observer on the pictures and its size calculated by an analyzing software. Comparing baseline wound margin (e.g. day 0) and follow up (e.g. day 3 and 7) allows the calculation of the ratio at baseline (day 0) and current situation (Figure 3).

Signs of inflammation/infection are assessed at each visit regarding to erythema, pain, odor, non-healing, excessive exudation and hyperthermia. Potential signs for allergic reactions are documented as well (erythema, edema and itching). Signs of inflammation/infection and/or signs of allergic skin reactions with a score >0 are reported as adverse events (AEs) irrespective of the treatment procedure (investigational product vs. standard plaster). Additionally, AEs and changes in concomitant medication are recorded.

In case of strong signs for allergic reaction or inflammation/infection, the intervention has been stopped. Last observation has to be carried forward for analysis and follow-up for safety reasons as long as the reaction persists (safety population SP).

The primary objective is the comparison of the investigational product versus traditional treatment (standard plaster) relating to the wound area assessed by quantitative image analysis at day 3 and day 7, quantified as area under the curve (AUC) and relative differences to baseline over time. The statistical analysis has been done on the AUC values and was based on the FAS (Full Analysis Set). FAS includes all subjects who do not violate any inclusion or exclusion criteria at the beginning of the trial, who receive at least one application of the test products and for whom at least one post-baseline assessment is performed. Safety parameters are documented and analyzed based on the SP (Safety Population; all subjects who received at least one application).

In conclusion, this novel wound healing model addresses three major objectives, to investigate
1. a routine approach with standardized incisions set and utilized as acute or non-acute wound model
2. the healing properties and relative wound healing rate of a topical wound healing approach in comparison to traditional or standard approaches as control (traditional treatment)
3. the outcome of acute and non-acute wounds treated with the different approaches

### References

[1] National Health System - Northamptonshire Healthcare (2017). Guidelines for the assessment management of wounds. Retrieved from https://www.nhft.nhs.uk/download.cfm?doc=docm93jijm4n1793
[2] Australian Research Society (2019). Wound care evidence based guidelines summary. Retrieved from https://www.research.qut.au.html
[3] Deutsche Gesellschaft für Kinderchirurgie e. V. (2013). AWMF Leitlinien-Register 006-129 Leitlinie zu Wunden und Wundbehandlung. Retrieved from https://www.awmf.org/leitlinien/detail/ll/006-129.html
[4] Deutsche Gesellschaft für Wundheilung und Wundbehandlung e. V. (2012). AWMF Leitlinien-Register 091/001S3-Leitlinie Lokaltherapie chronischer Wunden bei den Risiken CVI, PAVK und Diabetes mellitus. Retrieved from http://www.awmf.org/leitlinien/detail/ll/091-001.html
[5] Heyer K, Augustin M, Protz K, Herberger K, Spehr C, Rustenach SJ (2013). Effectiveness of advanced versus conventional wound dressings on healing of chronic wounds: systematic review and meta-analysis. Dermatology. 226 (2): 172-84. Retrieved from https://www.ncbi.nlm.nih.gov/pubmed/23711429
[6] MediGel®. Information for Use (July 2016). Retrieved from www.medigel.de
[7] Davis S, Gil J, Valdes J, Eberlein T (2013). Evaluating the efficacy of various over the counter topical formulations for stimulating epithelialization in a porcine partial thickness wound model. EWMA Kopenhagen Nov. 2013.
[8] Eberlein T, Gerke P, Lorenz H, Ammer R (2016). Advantages in wound healing by a topical easy to use wound healing lipo-gel for abrasive wounds-Evidence from a randomized, controlled experimental clinical study. Wound Medicine. Retrieved from https://doi.org/10.1016/j.wndm.2016.09.003
[9] Martin P, Nunan R (2015). Cellular and molecular mechanisms of repair in acute and chronic wound healing. Br J Dermatol. 173 (2): 370-378. Retrieved from https://www.ncbi.nlm.nih.gov/pmc/articles/PMC4671308/
[10] Wigger-Alberti W, Kuhlmann M, Ekanayake S, Wilhelm D (2009) Using a novel wound model to investigate the healing properties of products of superficial wounds. J Wound Care 18 (3): 123-128. Retrieved from https://www.ncbi.nlm.nih.gov/pubmed/19247233
[11] Gottrup, F, Agren, MS and Karlsmark, T (2000). Models for use in wound healing research: a survey focusing on in vitro and in vivo adult soft tissue. Wound Repair Regen 8: 83-96. Retrieved from https://www.ncbi.nlm.nih.gov/pubmed/10810034
[12] Wilhelm K-P, Wilhelm D, Bielfeldt S (2016) Models of wound healing: an emphasis on clinical studies. Skin Res Technol. doi: 10.1111/srt.12317. Retrieved from https://www.ncbi.nlm.nih.gov/pubmed/27503009
[13] Seldinger S (1953). Catheter replacement of the needle in percutaneous arteriography; a new technique. Acta radiol. 39 (5): 368-76. Retrieved from https://www.ncbi.nlm.nih.gov/pubmed/13057644
[14] Conz PA, Dissegna D, Rodighiero MP, La Greca G (1997). Cannulation of the internal jugular vein: comparison of the classic Seldinger technique and an ultrasound guided method. J Nephrol. 10 (6): 311-3. Retrieved from https://www.ncbi.nlm.nih.gov/pubmed/9442443
[15] Eming SA, Martin P, Tomic-Canic M (2014). Wound repair and regeneration: Mechanisms, signaling, and translation. Science translational medicine. 6 (265): 265. Retrieved from https://www.ncbi.nlm.nih.gov/pmc/articles/PMC4973620/
[16] EUnetHTA (2013). Retrieved from https://www.eunethta.eu/methodological-guideline-for-rea-of-pharmaceuticals-direct-and-indirect-comparison/
[17] Frodin T, Skogh M (1984). Measurement of transdermal water loss using an evaporimeter to follow the restitution of the barrier layer of human epidermis after stripping the stratum corneum. Acta Derm Venereol 64: 537-40. Retrieved from https://www.ncbi.nlm.nih.gov/pubmed/6084928
[18] Fitzpatrick Classification of Skin Types I through VI. Published in: Fitzpatrick T (1988). The Validity and Practicality of Sun-Reactive Skin Types I Through VI. Arch Dermatol. 124 (6): 869-871. Retrieved from https://jamanetwork.com/journals/jamadermatology/article-abstract/549509
[19] Descôteaux J (2007). Statistical power: An historical introduction. Tutorials in Quantitative Methods for Psychology. 3 (2): 28-34.
[20] Larsen HF, Ahlström MG, Gjerdrum LMR, Mogensen M, Ghathian K, Calum H, Sørensen AL, Forman JL, Vandeven M, Holerca MN, Du-Thumm L, Jorgensen LN, Agren MS (2017). Noninvasive measurement of reepithelialization and microvascularity of suction-blister wounds with benchmarking to histology. Wound Repair Regeneration. 25 (6): 984-993. Retrieved from https://www.ncbi.nlm.nih.gov/pubmed/29316016
[21] Ahlström MG, Gjerdrum LMR, Larsen HF, Fuchs C, Sørensen AL, Forman JL, Agren MS, Mogensen M (2018). Suction blister lesions and epithelialization monitored by optical coherence tomography. Skin Research and Technology. 24 (1): 65-72. Retrieved from https://www.ncbi.nlm.nih.gov/pubmed/28685861
[22] Wilhelm KP, Wilhelm D, Bielfeldt S (2017). Models of wound healing: an emphasis on clinical studies. Skin Research and Technology. 23 (1): 3-12. Retrieved from https://www.ncbi.nlm.nih.gov/pubmed/27503009

## Claims

1. A method for evaluating topical approaches applied on acute and none-acute wounds for their wound healing properties, comprising
a first step of evaluating a wound,
a second step of evaluating the wound after one to three days and after four to seven days and optionally after eight to fourteen days after the first step,
wherein the evaluation of the wound includes taking at least one digital photograph of the wound, optionally wherein a topical approach was applied to the wound during the time between each evaluation,
a third step of analyzing the photographs taken in the first and second step by quantitative image analysis,
wherein the quantitative image analysis includes determining the mean open wound area of the wound.

2. The method for evaluating topical approaches on wound healing properties according to claim 1,
further comprising a fourth step of analyzing wound healing by relating the mean open wound area to the time of each evaluation of the wound in the first and second step,
optionally wherein the fourth step further includes calculating a ratio of the mean open wound area of the wound of the evaluation of the first step to the mean open wound area of the wound of each evaluation of the wound in the first and second step.

3. The method for evaluating topical approaches on wound healing properties according to claim 1 or 2,
wherein the wound is an incision affecting all skin layers.

4. The method for evaluating topical approaches on wound healing properties according to any one of claims 1 to 3,
wherein the wound is caused by the procedure of a placement of a catheter, optionally wherein the catheter is placed using Seldinger technique,
wherein in the first step, the wound is evaluated after displacement of the catheter.

5. The method for evaluating topical approaches on wound healing properties according to claim 4,
wherein the wound is an acute wound caused by a cut conducted for the placement of a catheter, wherein the catheter is displaced after 1 to 24 hours, optionally after 2 to 12 hours, preferably after 4 to 6 hours after its placement, or
wherein the wound is a non-acute wound caused by a cut conducted for the placement of a catheter, wherein the catheter is displaced after 2 to 20 days, optionally after 6 to 18 days, optionally after 12 to 16 days, preferably after 14 days after its placement.

6. The method for evaluating topical approaches on wound healing properties according to claim 4 or 5,
wherein the catheter is an external multi-lumen catheter, optionally a Sheldon multi-lumen catheter with 1 mm to 6.67 mm diameter, preferably wherein the catheter is a Sheldon multi-lumen catheter with 3.67 mm diameter.

7. The method for evaluating topical approaches on wound healing properties according to any one of claims 1 to 6,
wherein the topical approach corresponds to any one of covering the wound with a plaster and/or covering the wound with a lipogel.

8. The method for evaluating topical approaches on wound healing properties according to any one of claims 1 to 7,
wherein the evaluation of the wound further includes the assessment of inflammation/infection regarding at least one of erythema, pain, odor, non-healing, excessive exudation and hyperthermia, and/or
wherein the evaluation of the wound further includes the assessment of allergic reactions regarding erythema, edema and itching.

9. The method for evaluating topical approaches on wound healing properties according to any one of claims 1 to 8,
wherein the area captured by the digital photographs is 0.3 to 20 cm in diameter.

10. The method for evaluating topical approaches on wound healing properties according to any one of claims 1 to 9,
wherein the mean open wound area is determined by digital microscopy, optionally including a histological image analysis program for measuring wound areas.

11. The method for evaluating topical approaches on wound healing properties according to any one of claims 1 to 10,
wherein in the second step, the wound is evaluated after three days and after seven days after the first step.

12. The method for evaluating topical approaches on wound healing properties according to any one of claims 4 to 11,
wherein the catheter is placed in the left or right external jungular vein in the neck.

13. The method for evaluating topical approaches on wound healing properties according to any one of the preceding claims,
wherein the evaluation comprises a comparison of the wound healing properties of an investigational product versus a standard product (for example a standard plaster).
